# EUROPÄISCHE PATENTSCHRIFT

(11) **EP 3 165 782 B1**
(45) Veröffentlichungstag und Bekanntmachung des Hinweises auf die Patenterteilung: **24.04.2019**
(21) Anmeldenummer: 15193023.7
(22) Anmeldetag: 04.11.2015
(51) Int. Cl.: F16C 33/04, A61M 1/10, A61M 1/12, C22C 29/06, C22C 29/12, C22C 29/16, B22F 7/06, F16C 17/02, F16C 17/08

(54) **CERMET-LAGER, INSBESONDERE FÜR EIN IMPLANTIERBARES MEDIZINISCHES GERÄT**
CERMET BEARING, IN PARTICULAR FOR AN IMPLANTABLE MEDICAL DEVICE
SUPPORT EN CERMET, EN PARTICULIER POUR UN APPAREIL MEDICAL IMPLANTABLE

(43) Veröffentlichungstag der Anmeldung: 10.05.2017
(73) Patentinhaber: Heraeus Deutschland GmbH & Co. KG, 63450 Hanau (DE)
(72) Erfinder: Gebert, Jörg-Martin, 76227 Karlsruhe (DE); Hausch, Ulrich, 60318 Frankfurt (DE); Schibli, Stefan, 60326 Frankfurt (DE)
(74) Vertreter: Herzog, Fiesser & Partner Patentanwälte PartG mbB

(56) Entgegenhaltungen:
- DE-A1-102009 035 971
- DE-A1-102010 006 689
- JP-A- 2004 003 633
- JP-A- 2004 330 455

## Beschreibung

Die Erfindung bezieht sich auf ein mechanisches Lager beinhaltend ein erstes Bauteil und ein weiteres Bauteil, wobei das mechanische Lager so ausgestaltet ist, dass das erste Bauteil und das weitere Bauteil gegeneinander eine Lagerbewegung ausführen können, wobei das erste Bauteil oder das weitere Bauteil oder beide ein Cermet beinhaltet. Ferner betrifft die Erfindung ein das mechanische Lager beinhaltendes implantierbares medizinisches Gerät, insbesondere eine Blutpumpe; sowie eine Verwendung eines Cermets zum Herstellen eines mechanischen Lagers; und eine Verwendung des mechanischen Lagers zum Lagern eines Bauteils eines implantierbaren medizinischen Geräts.

Die Erfindung betrifft insbesondere ein Herzunterstützungssystem, auch kurz Herzpumpe genannt. Im Stand der Technik wird eine Herzpumpe eingesetzt, um ein zu schwaches Herz beim Pumpen des Bluts zu unterstützen. Hierzu wird die Herzpumpe in den Körper des Patienten implantiert und über blutführende Schläuche mit der Aorta und dem Herzen verbunden. Die Herzpumpe beinhaltet einen Impeller, welcher eine Verstärkung der Blutströmung induziert. Der Impeller wird von einem Elektromotor angetrieben. Eine außerhalb des Körpers mitgeführte Steuereinheit sowie Batterien zur Energieversorgung sind über ein Stromkabel, welches durch die Haut des Patienten führt mit der Herzpumpe verbunden. Steuereinheit und Batterien können an einem Gürtel oder an Schulterholstern mitgeführt werden. Der Impeller der Herzpumpe kann mit der Motorwelle verbunden sein. Zur Lagerung der Motorwelle und damit des Impellers werden im Stand der Technik verschiedene Werkstoffe eingesetzt, welche jeweils spezifische Nachteile mit sich bringen.

Im Stand der Technik werden beispielsweise keramische Werkstoffe als Materialien eines Gleitlagers eingesetzt. Solche keramischen Werkstoffe haben eine geringe Wärmeleitfähigkeit. Dies kann in dem Gleitlager der Herzpumpe zu lokalen Erwärmungen und somit zu erhöhtem Verschleiß führen. Ferner kann es durch derartige lokale Erwärmungen zum Gerinnen des Bluts des Patienten kommen. Dies kann zum Tod des Patienten führen. Weiter werden im Stand der Technik Metalle als Gleitlagermaterialien in Herzpumpen eingesetzt. Hierbei weisen Metalle jedoch eine eher geringe Verschleiß festigkeit auf. Ferner werden im Stand der Technik beschichtete Gleitlagerkomponenten in Herzpumpen verwendet. Beim Einsatz von beschichteten Komponenten, insbesondere in einem mechanischen Lager, besteht stets ein Restrisiko eines Abblätterns der Beschichtung. Abgeblätterte Beschichtungsteile, die in den Körper des Patienten freigesetzt werden, können ebenfalls zu seinem Tod führen. Im Stand der Technik weiter bekannt ist der Einsatz von Steinen wie Rubin als Lagermaterial. Diese haben eine schlechte Wärmeleitfähigkeit und somit gilt auch hier das zu keramischen Werkstoffen über lokale Erwärmungen Gesagte. Graphit, welches im Stand der Technik ebenfalls als Lagermaterial bekannt ist, zeigt eine geringe Härte und somit einen großen Verschleiß. Gleiches gilt für Kunststoffe als Lagermaterialien. Auch gesinterte Gleitlager sind im Stand der Technik bekannt. Gesinterte, oftmals poröse Gleitlager ergeben im Einsatz meist eine Mischreibung, da Flüssigkeit sich in den Poren des gesinterten Werkstoffs ablagern kann und diese somit als Schmiermittel dient. Wird ein solches Lager in einer Herzpumpe eingesetzt ist die Flüssigkeit das Blut. Wenn sich Blut in den Poren ablagert, kann es verklumpen und Gewebe um das Lagermaterial wachsen, was sich nachteilig auf die Funktion der Herzpumpe und auf den Patienten auswirken kann. Im Stand der Technik werden in Herzpumpen ferner Magnetlager mit Permanentmagneten (DE 11 2006 002 413 T5) oder auch kombinierte magnetischmechanische Lager (WO 98/00185 A1, DE 196 25 300 A1) verwendet. Solche magnetischen Lager zeigen einen relativ geringen Verschleiß, sind jedoch sehr aufwendig herzustellen. Ferner kann das magnetische Feld der Permanentmagnete des Lagers sich negativ auf andere elektromagnetische Geräte auswirken. Ferner kann das präzise justierte magnetische Feld des Lagers durch äußere elektromagnetische Felder gestört werden, was zur Dejustierung des Magnetlagers führen kann.

JP 2004 330455 A offenbart ein mechanisches Lager beinhaltend ein erstes Bauteil und ein weiteres Bauteil, wobei das mechanische Lager so ausgestaltet ist, dass das erste Bauteil und das weitere Bauteil gegeneinander eine Lagerbewegung ausführen können, wobei ein Bauteil ein Cermet beinhaltet.

Allgemein ist es eine Aufgabe der vorliegenden Erfindung, einen Nachteil, der sich aus dem Stand der Technik ergibt, zumindest teilweise zu überwinden. Eine Aufgabe der Erfindung ist es, eine Herzpumpe bereitzustellen, welche implantiert in einem Patienten im Betrieb zu einer geringeren Thrombenbildung führen kann. Eine weitere Aufgabe der Erfindung ist es, ein mechanisches Lager bereitzustellen, welches gekennzeichnet ist durch eine möglichst vorteilhafte Kombination aus einer geringen Reibung, einer großen Verschleiß festigkeit und einer hohen Wärmeleitfähigkeit. Eine weitere Aufgabe der Erfindung ist es, ein mechanisches Lager bereitzustellen, welches möglichst nur aus biokompatiblen Materialien besteht. Eine weitere Aufgabe der Erfindung ist es, ein mechanisches Lager bereitzustellen, welches möglichst keine Beschichtung von Lagerbestandteilen beinhaltet. Eine weitere Aufgabe der Erfindung ist es, ein mechanisches Lager bereitzustellen, welches gekennzeichnet ist durch eine Kombination der vorgenannten Merkmale. Eine weitere Aufgabe der Erfindung ist es ein implantierbares medizinisches Gerät, beispielsweise eine Herzpumpe oder eine Blutpumpe oder beides, mit einem mechanischen Lager, welches eine der vorgenannten Aufgaben löst, bereitzustellen. Hierbei ist durch das mechanische Lager vorzugsweise ein Aktuator oder ein Impeller beweglich gelagert.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ausführungsform 1 eines mechanischen Lagers 1 beinhaltend ein erstes Bauteil und ein weiteres Bauteil, wobei das mechanische Lager so ausgestaltet ist, dass das erste Bauteil und das weitere Bauteil gegeneinander eine Lagerbewegung ausführen können, wobei das erste Bauteil oder das weitere Bauteil oder beide ein Cermet beinhaltet, bevorzugt aus dem Cermet besteht.

In einer erfindungsgemäßen Ausführungsform 2 ist das mechanische Lager 1 nach der Ausführungsform 1 ausgestaltet, wobei das Cermet ein Metall zu mindestens 20 Gew.-%, bevorzugt mindestens 25 Gew.-%, bevorzugter mindestens 30 Gew.-%, jeweils bezogen auf das Gesamtgewicht des Cermets beinhaltet. Einige Cermets weisen einen maximalen Metallgehalt von bis zu 90 Gew.-%, oder bis zu 80 Gew.-% oder bis zu 70 Gew.-% auf.

In einer erfindungsgemäßen Ausführungsform 3 ist das mechanische Lager 1 nach der Ausführungsform 1 ausgestaltet, wobei das Cermet ein Metall zu einem Metallanteil beinhaltet, wobei der Metallanteil eine monoton steigende Funktion von einer Position auf einer Geraden, welche durch das Cermet verläuft, ist. Bevorzugt beinhaltet das erste Bauteil eine erste Lageroberfläche und das weitere Bauteil eine weitere Lageroberfläche, wobei bei einem Ausführen der Lagerbewegung die erste Lageroberfläche gegen die weitere Lageroberfläche reibt, wobei das weitere Bauteil das Cermet beinhaltet, wobei der Metallanteil des Cermets an der weiteren Lageroberfläche minimal ist. Bevorzugt ist ein Keramikanteil des Cermets an der weiteren Lageroberfläche maximal.

In einer erfindungsgemäßen Ausführungsform 4 ist das mechanische Lager 1 nach der Ausführungsform 1 ausgestaltet, wobei das erste Bauteil eine erste Lageroberfläche beinhaltet, wobei das weitere Bauteil eine weitere Lageroberfläche beinhaltet, wobei bei einem Ausführen der Lagerbewegung die erste Lageroberfläche gegen die weitere Lageroberfläche reibt, wobei das weitere Bauteil das Cermet beinhaltet, wobei das Cermet ein Metall zu einem Metallanteil beinhaltet, wobei der Metallanteil innerhalb des weiteren Bauteils eine monoton steigende Funktion von einem Abstand von der weiteren Lageroberfläche ist. Ein in diesem Zusammenhang bevorzugter Cermet ist ein Gradientencermet, also ein Cermet mit einem räumlichen Gradienten des Metallanteils. Hierbei ist der Metallanteil bevorzugt an der weiteren Lageroberfläche minimal.

In einer erfindungsgemäßen Ausführungsform 5 ist das mechanische Lager 1 nach der Ausführungsform 3 oder 4 ausgestaltet, wobei der Metallanteil ein Minimum in einem Bereich von 0 bis 50 Gew.-%, bevorzugt 0 bis 25 Gew.-%, bevorzugter 0 bis 10 Gew.-%, weiterhin bevorzugt in einem Bereich von 0 bis 4 Gew.-%, jeweils bezogen auf das Gewicht eines räumlichen Ausschnitts des Cermets hat. Da der Metallanteil mit dem Abstand von der weiteren Lageroberfläche monoton mehr wird, hat das Cermet das Minimum des Metallanteils bevorzugt an der weiteren Lageroberfläche. Demnach beinhaltet der räumliche Ausschnitt des Cermets bevorzugt die weitere Lageroberfläche.

In einer erfindungsgemäßen Ausführungsform 6 ist das mechanische Lager 1 nach einer der Ausführungsformen 3 bis 5 ausgestaltet, wobei der Metallanteil ein Maximum in einem Bereich von 50 bis 95 Gew.-%, bevorzugt von 75 bis 95 Gew.-%, bevorzugter von 90 bis 95 Gew.-%, jeweils bezogen auf das Gewicht eines räumlichen Ausschnitts des Cermets hat. Hier liegt der räumliche Ausschnitt bevorzugt maximal entfernt von der weiteren Lageroberfläche.

In einer erfindungsgemäßen Ausführungsform 7 ist das mechanische Lager 1 nach einer der Ausführungsformen 2 bis 6 ausgestaltet, wobei das Metall biokompatibel ist.

In einer erfindungsgemäßen Ausführungsform 8 ist das mechanische Lager 1 nach einer der vorhergehenden Ausführungsformen ausgestaltet, wobei das Cermet eines ausgewählt aus der Gruppe bestehend aus ZrO, Al₂O₃, WC, SiN, Kohlenstoff und AlN zu höchstens 80 Gew.-%, bevorzugt zu höchstens 75 Gew.-%, bevorzugter zu höchstens 70 Gew.-%, jeweils bezogen auf das Gesamtgewicht des Cermets beinhaltet. Ein bevorzugter Kohlenstoff ist hierbei Diamant. Vorzugsweise sind die vorstehenden Cermets dotiert, wobei die Dotierung bevorzugt in einem Bereich von 0,0001 bis 5 Gew.-% und besonders bevorzugt in einem Bereich von 0,001 bis 1 Gew.-%, jeweils bezogen auf den Cermet liegt. Bevorzugte Dotierungsmittel sind Übergangsmetalle und seltene Erden, vorzugsweise ausgewählt aus der Gruppe bestehend aus Y, Ce, La, Nd, Sb, Ti oder mindestens zwei davon.

In einer erfindungsgemäßen Ausführungsform 9 ist das mechanische Lager 1 nach einer der vorhergehenden Ausführungsformen ausgestaltet, wobei das erste Bauteil oder das weitere Bauteil oder beide eines oder mehrere der folgenden Kriterien erfüllt:
a) eine Härte in einem Bereich von 400 bis 3500 HV;
b) eine spezifische Wärmeleitfähigkeit in einem Bereich von 30 bis 800 W/(m·K);
c) beinhaltet keine Beschichtung;
d) eine Porosität von weniger als 0,05, vorzugsweise weniger als 0,03 und besonders bevorzugt weniger als 0,01.

Hierbei ist es besonders bevorzugt, dass das erste Bauteil oder das weitere Bauteil oder beide eine der folgenden Merkmalskombinationen erfüllt a)b)c)d), a), b), c), d), a)b), a)b)d). Unter a) ist die Härte bevorzugt in einem Bereich von 400 bis 600 HV, bevorzugter von 430 bis 570 HV, bevorzugter von 470 bis 530 HV. Weiterhin besonders bevorzugt ist die Härte in einem Bereich von 430 bis 700 HV, bevorzugter von 430 bis 670, bevorzugter von 470 bis 630 HV, am bevorzugtesten von 480 bis 620 HV. Weiterhin ganz besonders bevorzugt ist die Härte in einem Bereich von 1000 bis 3500 HV, bevorzugter von 1500 bis 3300, bevorzugter von 1500 bis 3300 HV, am bevorzugtesten von 1800 bis 3200 HV. Unter b) ist die spezifische Wärmeleitfähigkeit bevorzugt in einem Bereich von 40 bis 60 W/(m·K), bevorzugter von 43 bis 57 W/(m·K), bevorzugter von 47 bis 53 W/(m·K). Weiterhin besonders bevorzugt ist die spezifische Wärmeleitfähigkeit in einem Bereich von 33 bis 55 W/(m·K), bevorzugter von 37 bis 50 W/(m·K), bevorzugter von 38 bis 47 W/(m·K). Weiterhin ganz besonders bevorzugt ist die spezifische Wärmeleitfähigkeit in einem Bereich von 500 bis 800 W/(m·K), bevorzugter von 550 bis 780 W/(m·K), bevorzugter von 580 bis 770 W/(m·K)

In einer erfindungsgemäßen Ausführungsform 10 ist das mechanische Lager 1 nach einer der vorhergehenden Ausführungsformen ausgestaltet, wobei das mechanische Lager einen Gleitreibungskoeffizienten in einem Bereich von 0,001 bis 10, bevorzugt von 0,01 bis 5, bevorzugter von 0,05 bis 0,25, hat.

In einer erfindungsgemäßen Ausführungsform 11 ist das mechanische Lager 1 nach einer der vorhergehenden Ausführungsformen ausgestaltet, wobei das mechanische Lager so ausgestaltet ist, dass die Lagerbewegung eines ausgewählt aus der Gruppe bestehend aus ein Freiheitsgrad, zwei Freiheitsgrade und drei Freiheitsgrade oder eine Kombination aus mindestens zwei davon hat.

In einer erfindungsgemäßen Ausführungsform 12 ist das mechanische Lager 1 nach einer der vorhergehenden Ausführungsformen ausgestaltet, wobei das mechanische Lager eines ausgewählt aus der Gruppe bestehend aus einem Gleitlager, einem Walzenlager, und einem Kugellager, oder eine Kombination aus mindestens zwei davon ist. Bevorzugt ist ein Gleitlager.

In einer erfindungsgemäßen Ausführungsform 13 ist das mechanische Lager 1 nach einer der vorhergehenden Ausführungsformen ausgestaltet, wobei das mechanische Lager ein Radiallager oder ein Linearlager oder beides ist. Bevorzugt ist ein Radiallager.

In einer erfindungsgemäßen Ausführungsform 14 ist das mechanische Lager 1 nach einer der vorhergehenden Ausführungsformen ausgestaltet, wobei das erste Bauteil eine Welle oder eine Achse oder beides, vorzugsweise eine Achse, ist. Hierbei ist eine Welle dazu ausgebildet ein Drehmoment zu übertragen. Bevorzugt ist eine Welle dadurch gekennzeichnet, dass sie ein Bauteil mit einer Antriebsfunktion ist. Ferner ist eine Achse dadurch gekennzeichnet, dass sie ein Bauteil zum Tragen oder Lagern oder beides von drehbaren Bauteilen ist. Eine bevorzugte Achse hat keine Antriebsfunktion.

In einer erfindungsgemäßen Ausführungsform 15 ist das mechanische Lager 1 nach einer der Ausführungsformen 1 bis 13 ausgestaltet, wobei das erste Bauteil eine Welle ist, wobei die Welle durch ein magnetisches Feld angetrieben wird. Bevorzugt wird die Welle durch eine Antriebseinheit, bevorzugt ein Elektromotor, berührungslos angetrieben.

Einen Beitrag zur Erfüllung mindestens einer der erfindungsgemäßen Aufgaben leistet eine Ausführungsform 1 eines implantierbaren medizinischen Geräts 1 beinhaltet das mechanische Lager 1 nach einer der Ausführungsformen 1 bis 15.

In einer erfindungsgemäßen Ausführungsform 2 ist das implantierbare medizinische Gerät 1 nach der Ausführungsform 1 ausgestaltet, wobei das implantierbare medizinische Gerät einen Motor beinhaltet. Bevorzugt ist der Motor mindestens teilweise hermetisch dicht eingehaust.

In einer erfindungsgemäßen Ausführungsform 3 ist das implantierbare medizinische Gerät 1 nach der Ausführungsform 1 oder 2 ausgestaltet, wobei das mechanische Lager einen Impeller lagert.

In einer erfindungsgemäßen Ausführungsform 4 ist das implantierbare medizinische Gerät 1 nach einer der Ausführungsformen 1 bis 3 ausgestaltet, wobei das implantierbare medizinische Gerät eine Pumpe oder ein Aktuator oder beides ist. Eine bevorzugte Pumpe ist eine Blutpumpe oder eine Herzpumpe oder beides.

Einen Beitrag zur Erfüllung mindestens einer der erfindungsgemäßen Aufgaben leistet eine Ausführungsform 1 eines Verfahrens 1 beinhaltend als Verfahrensschritte:
a) Bereitstellen eines Gehäuses; und
b) Einbringen eines beweglichen Bauteils in das Gehäuse;
wobei das Gehäuse aus einem biokompatiblen Material besteht, wobei das bewegliche Bauteil in dem Gehäuse durch das mechanische Lager 1 nach einer seiner Ausführungsformen 1 bis 15 beweglich gelagert ist. Ein bevorzugtes bewegliches Bauteil ist ein Impeller.

Einen Beitrag zur Erfüllung mindestens einer der erfindungsgemäßen Aufgaben leistet eine Ausführungsform 1 einer Vorrichtung 1 erhältlich durch das Verfahren 1 nach seiner Ausführungsform 1. Eine bevorzugte Vorrichtung ist ein implantierbares medizinisches Gerät.

Einen Beitrag zur Erfüllung mindestens einer der erfindungsgemäßen Aufgaben leistet eine Ausführungsform 1 eines Verfahrens 2 beinhaltend als Verfahrensschritte:
a) Bereitstellen des implantierbaren medizinischen Geräts 1 nach einer seiner Ausführungsformen 1 bis 4, oder der Vorrichtung 1 nach seiner Ausführungsform 1; und
b) Einbringen des implantierbaren medizinischen Geräts 1 oder der Vorrichtung 1 in einen eukaryotischen Organismus.

Einen Beitrag zur Erfüllung mindestens einer der erfindungsgemäßen Aufgaben leistet eine Ausführungsform 1 einer Verwendung 1 eines Cermets zum Herstellen eines mechanischen Lagers.

Einen Beitrag zur Erfüllung mindestens einer der erfindungsgemäßen Aufgaben leistet eine Ausführungsform 1 einer Verwendung 2 des mechanischen Lagers nach einer seiner Ausführungsformen 1 bis 15 zum Lagern eines Bauteils eines implantierbaren medizinischen Geräts.

Einen Beitrag zur Erfüllung mindestens einer der erfindungsgemäßen Aufgaben leistet eine Ausführungsform 1 einer Verwendung 3 des implantierbaren medizinischen Geräts 1 nach einer seiner Ausführungsformen 1 bis 4, oder der Vorrichtung 1 nach seiner Ausführungsform 1 zu einem Behandeln oder Therapieren eines eukaryotischen Organismus. Eine bevorzugte Behandlung oder Therapie ist eine solche einer Herzinsuffizienz. Bevorzugt ist hierbei eine Herzinsuffizienz mit Pumpversagen. Besonders bevorzugt sind Herzinsuffizienzen der Klassen III und IV, ganz besonders bevorzugt der Klasse IV, nach der Klasseneinteilung der New York Heart Association (NYHA).

Bevorzugte Ausgestaltungen von Bestandteilen einer erfindungsgemäßen Kategorie, insbesondere des erfindungsgemäßen mechanischen Lagers, des erfindungsgemäßen implantierbaren medizinischen Geräts, und des erfindungsgemäßen Verfahrens, sind ebenso bevorzugt für gleichnamige oder entsprechende Bestandteile der jeweils anderen erfindungsgemäßen Kategorien.

### mechanisches Lager

Bei einem mechanischen Lager entsteht durch die Lagerbewegung eine mechanische Reibung, bevorzugt eine Gleitreibung oder eine Rollreibung oder beides. Darüber hinaus muss das erfindungsgemäße mechanisches Lager nicht rein mechanisch sein, insbesondere kann das erfindungsgemäße mechanische Lager ein kombiniert mechanisch-magnetisches Lager sein. Hierzu kann das Lager einen oder mehrere Permanentmagneten, bevorzugt mindestens zwei gegenpolig zueinander ausgerichtete Permanentmagneten, beinhalten.

### Metall

Hier kommen alle dem Fachmann geläufigen Metalle in Betracht, die eine gute Verträglichkeit mit eukaryotischem Gewebe aufweisen. Ein erfindungsgemäß bevorzugtes Metall ist vorzugsweise ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder eine Kombination von mindestens zwei davon. Dabei ist eine bevorzugte Kombination eine Legierung. Ein bevorzugter Edelstahl ist ein Edelstahl 316L. Ein bevorzugtes Metall ist biokompatibel. Eine bevorzugte Legierung ist biokompatibel. Ein bevorzugtes biokompatibles Metall ist eines ausgewählt aus der Gruppe bestehend aus Palladium, Rhodium, Ruthenium, Molybdän, Platin, Iridium, Wolfram, Gold, Titan, Niob und Tantal oder eine Kombination aus mindestens zwei davon.

### Cermet

Erfindungsgemäß wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden, welches beispielsweise mindestens mit einem Bindemittel und ggf. mindestens mit einem Lösungsmittel versetzt werden kann. Das bzw. die keramischen Pulver des Cermets weisen vorzugsweise eine mittlere Korngröße von weniger als 10 µm, bevorzugt weniger als 5 µm, besonders bevorzugt weniger als 3 µm auf. Das bzw. die metallischen Pulver des Cermets wiesen vorzugsweise eine mittlere Korngröße von weniger als 15 µm, bevorzugt weniger als 10 µm, besonders bevorzugt weniger als 5 µm auf. Als mittlere Korngröße wird dabei insbesondere der Medianwert oder D₅₀-Wert der Korngrößenverteilung angesehen. Der D₅₀-Wert beschreibt jenen Wert, bei dem 50 % der Körner des keramischen Pulvers und/oder des metallischen Pulvers feiner sind als der D₅₀-Wert. Ein bevorzugtes Cermet weist eine hohe spezifische Leitfähigkeit auf, die bevorzugt mindestens 1 S/m, bevorzugter mindestens 100 S/m, bevorzugter mindestens 10³ S/m, bevorzugter mindestens 10⁴ S/m , noch bevorzugter mindestens 10⁵ S/m, und am bevorzugtesten mindestens 10⁶ S/m beträgt.

Die mindestens eine keramische Komponente eines erfindungsgemäßen Cermets beinhaltet bevorzugt eine erfindungsgemäße Keramik. Die mindestens eine metallische Komponente eines erfindungsgemäßen Cermets beinhaltet bevorzugt eines ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder Kombination von mindestens zwei davon. Dabei ist eine bevorzugte Kombination eine Legierung. Ein bevorzugter Edelstahl ist ein Edelstahl 316L. Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu muss der Metallgehalt erfahrungsgemäß, abhängig von der Materialauswahl, mindestens 25 Vol.-% betragen, bevorzugt mindestens 32 Vol.-%, am bevorzugtesten mindestens 38 Vol.-%, jeweils bezogen auf das gesamte Volumen des Cermets. Bei einem besonders bevorzugten Cermet ist die keramische Komponente Diamant oder Al₂O₃ und die metallische Komponente Platin.

### Keramik

Eine erfindungsgemäße Keramik kann jede Keramik sein, die der Fachmann für den erfindungsgemäßen Einsatz auswählen würde. Die Keramik ist bevorzugt ausgewählt aus der Gruppe bestehend aus einer Oxidkeramik, einer Silikatkeramik, einer Nichtoxid-Keramik und einer Elementkeramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA - Zirconia Toughened Aluminum - Al₂O₃/ZrO₂), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AlN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

Die Elementkeramik ist bevorzugt Kohlenstoff, besonders bevorzugt Diamant. Zum Herstellen eines Cermets, welches als keramische Komponente eine Elementkeramik beinhaltet wird bevorzugt ein Diamantpulver mit einem Metallpulver gemischt. Ein solcher Cermet mit einer Elementkeramik als keramischer Komponente zeichnet sich durch eine besonders bevorzugte Kombination aus hoher Härte und hoher spezifische Wärmeleitfähigkeit aus.

### biokompatibles Material

Ein bevorzugtes biokompatibles Material ist eines ausgewählt aus der Gruppe bestehend aus biotolerant, bioinert und bioaktiv oder eine Kombination aus mindestens zwei davon.

### hermetisch dicht

Im Rahmen der Erfindung kann der Begriff "hermetisch dicht" verdeutlichen, dass bei einem bestimmungsgemäßen Gebrauch innerhalb üblicher Zeiträume (beispielsweise 5 bis 10 Jahre) Feuchtigkeit oder Gase oder beides nicht oder nur minimal durch die hermetisch dichte Barriere, beispielsweise das Gehäuse, hindurch zwischen Innen und Außen ausgetauscht werden können. Eine physikalische Größe, die beispielsweise eine Permeation von Gasen oder Feuchtigkeit oder beidem durch die Barriere beschreiben kann, ist die sogenannte Leckrate, welche beispielsweise durch Lecktests bestimmt werden kann. Entsprechende Lecktests können beispielsweise mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung, hier beispielsweise dem Innenvolumen des Gehäuses, festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von 1 x 10⁻⁸ atm×cm³/s bis 1 x 10⁻⁷ atm×cm³/s betragen. Im Rahmen der Erfindung kann der Begriff "hermetisch dicht" insbesondere bedeuten, dass die Barriere eine Helium-Leckrate von weniger als 1 x 10⁻⁷ atm×cm³/s aufweist. In einer vorteilhaften Ausführung kann die Helium-Leckrate weniger als 1 x 10⁻⁸ atm×cm³/s, insbesondere weniger als 1 x 10⁻⁹ atmcm³/s betragen. Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben. Aufgrund der Einsatzart von implantierbaren medizinischen Geräten ist die hermetische Dichtigkeit und Biokompatibilität der elektrischen Komponenten, beispielsweise des Motors, in der Regel eine der vorrangigsten Anforderungen. Das hier vorgeschlagene implantierbare medizinische Gerät kann insbesondere in einen Körper eines menschlichen oder tierischen Benutzers, insbesondere eines Patienten, eingesetzt werden. Dadurch ist das Gerät in der Regel einer Körperflüssigkeit wie Blut ausgesetzt. Somit ist es in der Regel von Bedeutung, dass weder Körperflüssigkeit in elektrische Komponenten eindringt, noch das Flüssigkeiten aus den elektrischen Komponenten austreten. Um dieses sicherzustellen, sollte die Einhausung der elektrischen Komponenten eine möglichst vollständige Undurchlässigkeit aufweisen, insbesondere gegenüber Körperflüssigkeiten.

### eukaryotischer Organismus

Ein bevorzugter eukaryotischer Organismus ist ein tierischer Organismus oder ein menschlicher Organismus oder beides.

### MESSMETHODEN

Die folgenden Messmethoden wurden im Rahmen der Erfindung benutzt. Sofern nichts anderes angegeben ist wurden die Messungen bei einer Umgebungstemperatur von 25°C, einem Umgebungsluftdruck von 100 kPa (0,986 atm) und einer relativen Luftfeuchtigkeit von 50 % durchgeführt.

### Biokompatibilität

Die Biokompatibilität wird gemäß der Norm ISO 10993-4:2002 bestimmt.

### Härte

Die Härte wurde gemäß der DIN EN ISO 6507-1 bis -4. gemessen. Hierzu wurde ein ZHVµ Mikro Vickers Härteprüfer der Firma Zwick Roell AG, Ulm, Deutschland sowie die dazugehörige Software verwendet. Als Eindringkörper wurde die Vickerspyramide 136° verwendet. Die gewählte Last liegt bei 2000g und die Haltezeit bei 15 s.

### spezifische Wärmeleitfähigkeit

Die Messung der spezifischen Wärmeleitfähigkeit erfolgte mit einer LFA 467 HyperFlash Light Flash-Apparatur der Firma NETZSCH-Gerätebau GmbH, Selb/Bayern, Deutschland. Für die Messung wurde ein zylindrischer Probenkörper mit Durchmesser 10 mm und Dicke 5 mm eingesetzt. Gemessen wurde in Vakuum sowie in einem Temperaturbereich von 10 bis 50°C.

### Gleitreibungskoeffizient

Der Gleitreibungskoeffizient bestimmt sich als µ = F_{R} / F_{N}, wobei F_{R} die Reibungskraft ist und F_{N} die Normalkraft. Die Bestimmung von µ erfolgt demnach über eine Messung eines Kraft-Weg-Verlaufes eines Reibvorgangs. Zunächst werden 2 Proben mit jeweils planparallelen Oberflächen aus dem zu untersuchenden Material hergestellt. Die Reibungsmessung erfolgt bei trockener Reibung. Ferner erfolgt die Messung mit einer Prüfmaschine Zwick Z0.5 der Zwick GmbH & Co. KG, Ulm sowie der zugehörigen Software. Der Messaufbau besteht aus einem einachsigen Motortisch und einem Schlitten. Die Gleitpartner werden am Motortisch und am Schlitten befestigt und gegen einander bewegt. Zuerst wird der Schlitten bis zum Ansprechen des Kraftsensors bewegt und dann wieder entlastet. Erst nach einer Ruhezeit erfolgt die eigentliche Aufzeichnung der Reibkraft F_{R}. Um Beschleunigungskräfte zu eliminieren, wird F_{R} nicht am Motortisch gemessen, sondern am unbeweglichen Schlitten. Zur Auswertung muss außerdem die Normalkraft F_{N} bekannt sein. Hier wird F_{N} über den Anpressdruck der beiden Proben zueinander auf einen Wert von 10 bis 50 N/mm² festgelegt. Für die Gleitreibkraft F_{R} wird der Mittelwert während einer festgelegten Wegstrecke herangezogen.

### Porosität

Zur Messung der Porosität wurden zunächst metallographische Schliffproben durch Einbetten in Epoxidharz, Schleifen mit SiC-Papier mit sukzessive kleinerer Körnung sowie Polieren mit einer Diamantpaste hergestellt. Die äußerste Schicht jeder Probe wurde durch Ätzen der Probe abgetragen. Im Folgenden wurden Aufnahmen der so behandelten Probenoberfläche mit einem Lichtmikroskop und einem Elektronenmikroskop gemacht. Hierbei sollt ein möglichst hoher Kontrast zwischen den Poren der Probe und dem Material (Metall und Keramik) erzielt werden. Zur Auswertung der Bilder wurden diese Graustufenbilder mittels Otsu-Methode in binäre Bilder umgewandelt. Das heißt, die Bildpixel wurden mittels eines Schwellenwertes jeweils einer Pore oder dem Probenmaterial zugeordnet. Anschließend wurde die Porosität an Hand der binären Bilder als Quotient aus der Anzahl der Pixel, welche Poren darstellen und der Gesamtanzahl der Pixel pro Bild ermittelt. Hierbei wurde die Porosität als arithmetischer Mittelwert aus 5 Bildern, jeweils aufgenommen an 5 Schliffproben, bestimmt.

### Thrombenbildung

Zur Bestimmung der Thrombenbildung in einer Herzpumpe wurde zunächst eine ausreichende Menge Blut (bis zu 21) aus einem Schwein (1 Tier je Test) entnommen. Mit diesem Blut wurde der In-Vitro-Test gemäß "In-vitro-Teststand für die Untersuchung der Thrombenbildung in Blutpumpen und anderen blutkontaktierten Systemen" (Biomedizinische Technik/Biomedical Engineering. Band 37, Heft s1, Seiten 266-268, 1992, ISSN (Online) 1862-278X, ISSN (Print) 0013-5585, DOI: 10.1515/bmte.1992.37.s1.266, Juli 2009 online erschienen) von H. Schima et al. durchgeführt. Hierbei wurde die jeweils zu untersuchende Herzpumpe in dem beschrieben Versuchsaufbau verwendet. Als Testzeitraum wurden 3 Stunden gewählt. Wurde gemäß der Testdurchführung festgestellt, dass die Blutabnahme fehlerhaft war, so wurde diese Testdurchführung verworfen. Nach der Testdurchführung gemäß der zitierten Quelle wurde die getestete Herzpumpe auseinandergebaut und die Einzelteile auf Thromben untersucht. Identifizierte Thromben wurden bezüglich ihrer Größe vermessen.

### BEISPIELE

Die Erfindung wird im Folgenden durch Beispiele und Zeichnungen genauer dargestellt, wobei die Beispiele und Zeichnungen keine Einschränkung der Erfindung bedeuten. Die Zeichnungen sind, sofern nicht anders angegeben, nicht maßstabsgetreu.

### erfindungsgemäßes Beispiel

Zunächst wurde eine Lagerpfanne eines mechanischen Lagers für den Impeller einer kommerziell erhältlichen Herzpumpe aus erfindungsgemäßen Materialien hergestellt. Hierfür kommen Herzpumpen wie HeartMate II der Thoratec Europe Limited, Cambridgeshire Großbritannien; HVAD (VAD - Ventricular Assist System) der HeartWare GmbH, Hannover, Deutschland; oder INCOR der Berlin Heart GmbH, Berlin, Deutschland in Frage. Hierbei ist der HeartMate II ein sehr verbreitetes Produkt.

Zur Herstellung der erfindungsgemäßen Lagerpfanne wurden zunächst Cermetpasten aus Al₂O₃, Platin und organischem Vehikel hergestellt. Hierbei wurden für die unterschiedlichen Cermetpasten verschiedene Mengen Platinpulver verwendet. Für die verschiedenen Cermetpasten wurden 3 bis 60 g Platinpulver jeweils mit 24 g Al₂O₃-Pulver und einem organischen Vehikel auf Ethylzellulosebasis gemischt und diese Mischung in einer 3-Walzenmühle homogenisiert. Die so erhaltenen Pasten hatten eine Viskosität im Bereich von 250 bis 500 Pa·s sowie eine Mahlfeinheit (fineness of grind - FoG) von weniger als 10 µm. Hierbei war darauf zu achten, dass die Pasten auf Grund ihrer rheologischen Eigenschaften (insbesondere Viskosität) zum Drucken geeignet waren.

Der detaillierte Pastenherstellungsprozess wird im Folgenden dargelegt. Die Komponenten wurden in einem Becherglas manuell gemischt. Diese vorgemischte Paste wurde in einer Exakt E80 (von EXAKT Advanced Technologies GmbH) 3-Walzenmühle mit Edelstahlrollen homogenisiert. Die Paste wurde der Mühle mehrfach zugeführt, wobei die Spalte (Spalt 1 zwischen der ersten und zweiten Rolle, Spalt 2 zwischen der zweiten und dritten Rolle) zwischen den Rollen reduziert wurden. Die Spalte konnten hierbei entweder über die Spaltbreite (Abstandskontrolle) oder über den Druck zwischen den Rollen (Druckkontrolle) geregelt werden. Die ersten Mahlschritte wurden mit Abstandskontrolle durchgeführt, die finalen Mahlschritte mit Druckkontrolle. Im Folgenden sind die Werte aufgeführt, welche in den einzelnen Mahlschritten verwendet wurden. Hierbei waren Abstand und Druck jeweils für Spalt 1 und 2 gleich.
Mahlschritte 1 und 2: Abstandskontrolle 20 µm
Mahlschritte 3 und 4: Abstandskontrolle 15 µm
Mahlschritte 5 und 6: Abstandskontrolle 10 µm
Mahlschritte 7 und 8: Abstandskontrolle 5 µm
Mahlschritte 9, 10 und 11: Druckkontrolle 10 N/mm
Mahlschritte 12, 13 und 14: Druckkontrolle 12.5 N/mm
Mahlschritte 15, 16 und 17: Druckkontrolle 15 N/mm

In weiteren erfindungsgemäßen Beispielen wurden kommerziell erhältliche Cermetpasten wie CL11-8822 (von Heraeus Precious Metals North America, Conshohocken LLC) verwendet.

Mittels 3D-Siebdruck wurde aus den Cermetpasten mit verschiedenen Platinanteilen ein Grünling hergestellt. Hierbei wurde so vorgegangen, dass die unterschiedlichen Pasten mit entsprechend unterschiedlichen Platin- und Al₂O₃-Anteilen in mehreren Lagen durch Siebdruck zu dem Grünling aufgebaut wurden. Hierbei wurde ein Gradient des Metallanteils in Richtung senkrecht zu den Lagen realisiert. Die Richtung des Gradienten wurde so gewählt, dass nahe an der Lageroberfläche der Lagerpfanne ein hoher Keramikanteil von 85 bis 95 Gew.-% und ca. 50 µm weiter in den Grünling hinein ein hinreichend hoher Metallanteil erreicht wurden. Der Übergang zwischen den unterschiedlichen Bereichen musste hier fließend ausgestaltet werden, damit keine Delamination auf Grund der unterschiedlichen Materialeigenschaften (z.B. thermische Ausdehnung) auftritt.

Der so erhaltene Grünling wurde bei ca. 1500 °C gesintert. Zur weiteren Reduzierung der Porosität auf unter 0,01 wurde das erhaltene Bauteil heiß-isostatisch gepresst. Durch anschließendes Schleifen und Polieren wurde das erhaltene Lagerteil passgerecht für die obige Herzpumpe bearbeitet. Die Lagerpfanne besteht zu 70 Gew.-% aus Platin und zu 30 Gew.-% aus Al₂O₃, jeweils bezogen auf das Gesamtgewicht der Lagerpfanne. Hierbei ist der Metallanteil an der Oberfläche der Lagerpfanne, welche die Welle gleitreibend lagert, minimal bei 5 Gew.-% und im maximalen Abstand von dieser Oberfläche maximal bei 90 Gew.-%.

Ferner wurde eine kommerziell erhältliche Herzpumpe bereitgestellt. Diese wurde geöffnet und das herkömmliche Lager entfernt. In dem Lager wurde die Lagerpfanne durch die gemäß den obigen Ausführungen hergestellte erfindungsgemäße Lagerpfanne ersetzt und das Lager wieder in die Herzpumpe eingebaut. Die so erhaltene Herzpumpe wurde anschließend Tests unterzogen.

### nicht erfindungsgemäße Vergleichsbeispiele

Hierfür wurde eine kommerziell erhältliche Herzpumpe mit dem herkömmlichen Lager getestet. Ferner wurden Lager aus Keramik, Metall, Kunststoff und Stein in dieser Herzpumpe getestet.

### Auswertung

Durch Vergleich des erfindungsgemäßen Beispiels mit den Vergleichsbeispielen zeigte sich, dass das erfindungsgemäße Lager in der Herzpumpe im Vergleich zu den nicht erfindungsgemäßen Lagern eine vorteilhafte Kombination aus geringer Reibung, hoher Verschleißfestigkeit und hoher Wärmeleitfähigkeit aufweist. Zudem besteht das erfindungsgemäße Lager aus biokompatiblen Materialien. Ferner wurde der oben beschriebene Test für die Thrombenbildung durchgeführt. Hier zeigte sich, dass Herzpumpen mit dem erfindungsgemäßen Lager weniger und kleinere Thromben bilden.

Es zeigen:
- Figur 1: eine schematische Querschnittsdarstellung eines erfindungsgemäßen mechanischen Lagers;
- Figur 2a): eine schematische Querschnittsdarstellung eines weiteren erfindungsgemäßen mechanischen Lagers;
- Figur 2b): eine schematische Querschnittsdarstellung eines weiteren erfindungsgemäßen mechanischen Lagers;
- Figur 3: eine schematische Querschnittsdarstellung eines weiteren erfindungsgemäßen mechanischen Lagers;
- Figur 4: eine schematische Querschnittsdarstellung eines erfindungsgemäßen implantierbaren medizinischen Geräts;
- Figur 5: eine schematische Darstellung eines linksventrikulären Herzunterstützungssystems mit einer erfindungsgemäßen Herzpumpe; und
- Figur 6: ein Ablaufdiagramm eines erfindungsgemäßen Verfahrens.

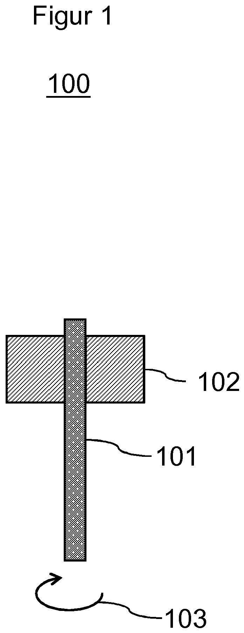

Figur 1 zeigt eine schematische Querschnittsdarstellung eines erfindungsgemäßen mechanischen Lagers 100. Das mechanische Lager 100 ist ein Gleitlager, genauer ein Radiallager. Das mechanische Lager 100 beinhaltend ein erstes Bauteil 101, welches eine Welle eines Elektromotors oder eine Achse ist, und ein weiteres Bauteil 102, welches eine Lagerschale ist. Das mechanische Lager 100 ist so ausgestaltet, dass die Welle / Achse 101 und die Lagerschale 102 gegeneinander eine Lagerbewegung 103, eine Rotation der Welle / Achse 101, ausführen können. Die Lagerschale 102 besteht aus einem Cermet. Das Cermet besteht zu 30 Gew.-% aus Titan und zu 70 Gew.-% aus Al₂O₃, jeweils bezogen auf das Gesamtgewicht des Cermets. Das Cermet ist biokompatibel.

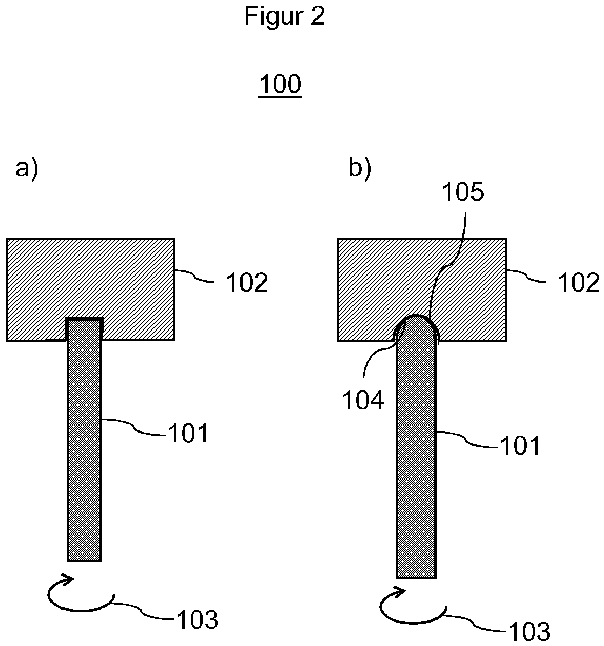

Figur 2a) zeigt eine schematische Querschnittsdarstellung eines weiteren erfindungsgemäßen mechanischen Lagers 100. Das mechanische Lager 100 ist ein Gleitlager, genauer ein Axiallager. Das mechanische Lager 100 beinhaltend ein erstes Bauteil 101, welches eine Welle eines Elektromotors oder eine Achse ist, und ein weiteres Bauteil 102, welches eine Lagerschale ist. Das mechanische Lager 100 ist so ausgestaltet, dass die Welle / Achse 101 und die Lagerschale 102 gegeneinander eine Lagerbewegung 103, eine Rotation der Welle / Achse 101, ausführen können. Die Lagerschale 102 besteht aus einem Cermet. Das Cermet besteht zu 25 Gew.-% aus Palladium und zu 75 Gew.-% aus ZrO, jeweils bezogen auf das Gesamtgewicht des Cermets. Das Cermet ist biokompatibel.

Figur 2b) zeigt eine schematische Querschnittsdarstellung eines weiteren erfindungsgemäßen mechanischen Lagers 100. Das mechanische Lager 100 ist ein Gleitlager, genauer ein Axiallager. Das mechanische Lager 100 beinhaltend ein erstes Bauteil 101, welches eine Welle eines Elektromotors oder eine Achse ist, und ein weiteres Bauteil 102, welches eine Lagerschale ist. Das mechanische Lager 100 ist so ausgestaltet, dass die Welle / Achse 101 und die Lagerschale 102 gegeneinander eine Lagerbewegung 103, eine Rotation der Welle / Achse 101, ausführen können. Bei dem Ausführen der Lagerbewegung reibt eine erste Lageroberfläche 104 der Welle 101 gegen eine weitere Lageroberfläche 105 der Lagerschale 102. Die Lagerschale 102 besteht aus einem Cermet mit einem räumlichen Gradienten eines Metallanteils. Das Metall ist hierbei Platin. Eine keramische Komponente des Cermets ist Al₂O₃. Der Metallanteil ist an der weiteren Lageroberfläche 0 Gew.-% bezogen auf das Gewicht eines räumlichen Ausschnitts des Cemets, der die weitere Lageroberfläche 105 beinhaltet und keinen Punkt innerhalb des Cermets beinhaltet, der weiter als 1 mm von der weiteren Lageroberfläche 105 entfernt ist. Der Metallanteil des Cermets wird mit einem Abstand von der weitere Lageroberfläche 105 mehr. Ein Maximum des Metallanteils in dem Cermet liegt bei 90 Gew.-% bezogen auf das Gewicht eines räumlichen Ausschnitts des Cermets.

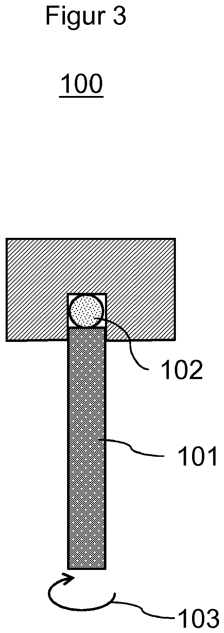

Figur 3 zeigt eine schematische Querschnittsdarstellung eines weiteren erfindungsgemäßen mechanischen Lagers 100. Das mechanische Lager 100 ist ein axiales Kugellager. Das mechanische Lager 100 beinhaltend ein erstes Bauteil 101, welches eine Welle eines Elektromotors oder eine Achse ist, und ein weiteres Bauteil 102, welches eine Kugel in einer Lagerschale ist. Das mechanische Lager 100 ist so ausgestaltet, dass die Welle / Achse 101 gegenüber der Kugel 102 und der Lagerschale eine Lagerbewegung 103, eine Rotation der Welle 101, ausführen kann. Die Kugel 102 und die Lagerschale bestehen jeweils aus einem Cermet. Das Cermet besteht zu 28 Gew.-% aus Titan und zu 72 Gew.-% aus AlN, jeweils bezogen auf das Gesamtgewicht des Cermets. Das Cermet ist biokompatibel.

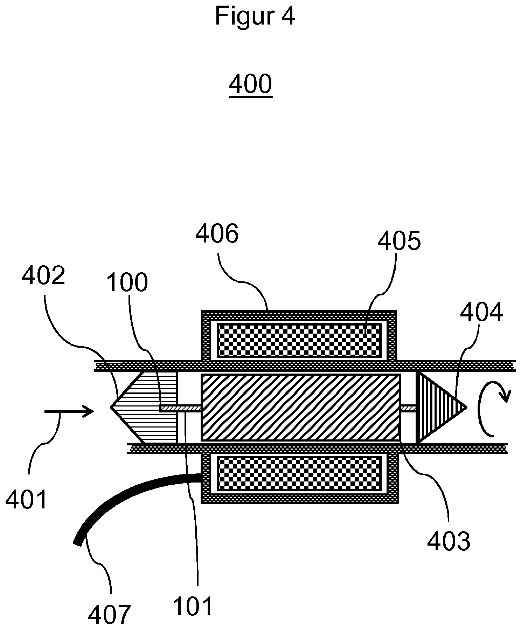

Figur 4 zeigt eine schematische Querschnittsdarstellung eines erfindungsgemäßen implantierbaren medizinischen Geräts 400, welches eine Herzpumpe ist. Blut, welches in Blutflussrichtung 401 in die Herzpumpe 400 strömt, passiert zunächst einen Strömungsgleichrichter 402, welcher Turbulenzen der Blutströmung vermindert. Eine Welle 101 trägt einen Impeller 403. Die Welle 101 und somit der Impeller 403 sind in einem erfindungsgemäßen Gleitlager 100 gelagert. Der Impeller verstärkt die Strömung des Bluts und unterstützt somit die Pumpfunktion des Herzens. Die Herzpumpe 400 beinhaltet weiter einen Diffusor 404. Angetrieben wird die Welle 101 durch einen Elektromotor, dessen Stator 405 hermetisch dicht in einem Gehäuse 406 eingehaust ist. Der Elektromotor treibt hierbei die Welle 101 berührungslos durch ein elektromagnetisches Feld an. Über ein Stromkabel 407 wird der Elektromotor mit Strom betrieben.

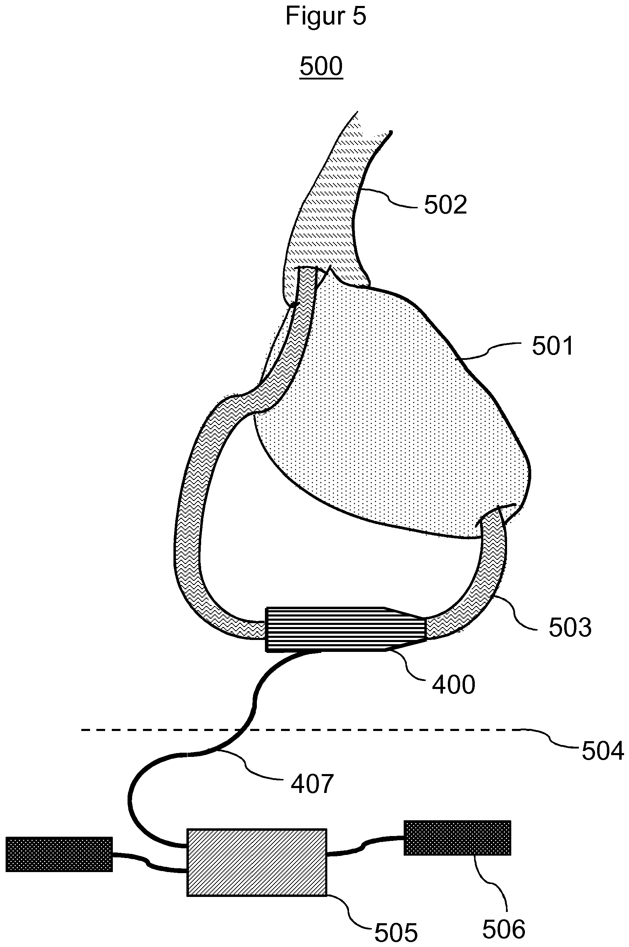

Figur 5 zeigt eine schematische Darstellung eines linksventrikulären Herzunterstützungssystems 500 mit einer implantierten erfindungsgemäßen Herzpumpe 400 nach Figur 4. Die Herzpumpe 400 ist über einen blutführenden Schlauch 503 an die Spitze des Herzens 501 und über einen weiteren blutführenden Schlauch 503 an die Aorta 502 angeschlossen. Ein Stromkabel 407, welches die Herzpumpe 400 mit Strom versorgt, verläuft von innerhalb des Körpers des Patienten durch eine Hautgrenze 504 nach außen zu einer Steuerungseinheit 505, welche die Herzpumpe 400 steuert. Die Steuereinheit 505 kann durch den Patienten an einem Gürtel getragen werden. Die Steuereinheit 505 ist durch weitere Stromkabel 407 mit zwei Batterien 506 verbunden. Die Batterien 506 können von dem Patienten in jeweils einem Schulterholster getragen werden.

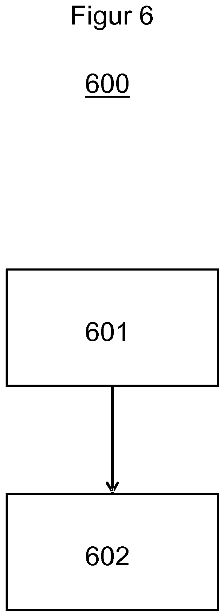

Figur 6 zeigt ein Ablaufdiagramm eines erfindungsgemäßen Verfahrens 600 zum Herstellen einer Herzpumpe 400. In einem Verfahrensschritt a) 601 wird ein Gehäuse aus Titan mit einem Strömungskanal bereitgestellt. In einem Verfahrensschritt b) 602 wird eine Welle / Achse 101 mit einem Impeller 402 in den Strömungskanal eingebracht. Die Welle / Achse 101 wird mit dem Radiallager nach Figur 1 in dem Strömungskanal gelagert.

### LISTE DER BEZUGSZEICHEN

- **100**: erfindungsgemäßes mechanisches Lager
- **101**: erstes Bauteil / Welle / Achse
- **102**: weiteres Bauteil
- **103**: Lagerbewegung
- **104**: erste Lageroberfläche
- **105**: weitere Lageroberfläche
- **400**: erfindungsgemäßes implantierbares medizinisches Gerät / Herzpumpe
- **401**: Blutflussrichtung
- **402**: Strömungsgleichrichter
- **403**: Impeller
- **404**: Diffusor
- **405**: Motorstator
- **406**: Gehäuse
- **407**: Stromkabel
- **500**: linksventrikuläres Herzunterstützungssystem
- **501**: Herz
- **502**: Aorta
- **503**: Schlauch
- **504**: Hautgrenze
- **505**: Steuereinheit
- **506**: Batterie
- **600**: erfindungsgemäßes Verfahren
- **601**: Verfahrensschritt a)
- **602**: Verfahrensschritt b)

## Patentansprüche

1. Ein mechanisches Lager (100) beinhaltend ein erstes Bauteil (101) und ein weiteres Bauteil (102),
wobei das mechanische Lager (100) so ausgestaltet ist, dass das erste Bauteil (101) und das weitere Bauteil (102) gegeneinander eine Lagerbewegung (103) ausführen können, wobei das erste Bauteil (101) oder das weitere Bauteil (102) oder beide ein Cermet beinhaltet,
wobei das Cermet ein Metall zu mindestens 20 Gew.-% bezogen auf das Gesamtgewicht des Cermets beinhaltet,
wobei das Metall biokompatibel ist.

2. Ein mechanisches Lager (100) beinhaltend ein erstes Bauteil (101) und ein weiteres Bauteil (102),
wobei das mechanische Lager (100) so ausgestaltet ist, dass das erste Bauteil (101) und das weitere Bauteil (102) gegeneinander eine Lagerbewegung (103) ausführen können, wobei das erste Bauteil (101) oder das weitere Bauteil (102) oder beide ein Cermet beinhaltet,
wobei das Cermet ein Metall zu einem Metallanteil beinhaltet,
wobei der Metallanteil eine monoton steigende Funktion von einer Position auf einer Geraden, welche durch das Cermet verläuft, ist.

3. Das mechanische Lager nach Anspruch 2, wobei der Metallanteil ein Minimum in einem Bereich von 0 bis 50 Gew.-% bezogen auf das Gewicht eines räumlichen Ausschnitts des Cermets hat.

4. Das mechanische Lager nach einem der Ansprüche 2 oder 3, wobei der Metallanteil ein Maximum in einem Bereich von 50 bis 95 Gew.-% bezogen auf das Gewicht eines räumlichen Ausschnitts des Cermets hat.

5. Ein implantierbares medizinisches Gerät (400) beinhaltet das mechanische Lager (100) nach einem der vorhergehenden Ansprüche.

6. Ein Verfahren (600) beinhaltend als Verfahrensschritte (601, 602):
a) Bereitstellen eines Gehäuses; und
b) Einbringen eines beweglichen Bauteils in das Gehäuse;
wobei das Gehäuse aus einem biokompatiblen Material besteht,
wobei das bewegliche Bauteil in dem Gehäuse durch das mechanische Lager (100) nach einem der Ansprüche 1 bis 4 beweglich gelagert ist.

7. Eine Vorrichtung erhältlich durch das Verfahren (600) nach Anspruch 6.

8. Eine Verwendung eines Cermets zum Herstellen eines mechanischen Lagers (100),
wobei das Cermet ein Metall zu mindestens 20 Gew.-% bezogen auf das Gesamtgewicht des Cermets beinhaltet,
wobei das Metall biokompatibel ist.

9. Eine Verwendung eines mechanischen Lagers (100) zum Lagern eines Bauteils eines implantierbaren medizinischen Geräts (400),
wobei das mechanische Lager (100) ein erstes Bauteil (101) und ein weiteres Bauteil (102) beinhaltet,
wobei das mechanische Lager (100) so ausgestaltet ist, dass das erste Bauteil (101) und das weitere Bauteil (102) gegeneinander eine Lagerbewegung (103) ausführen können, wobei das erste Bauteil (101) oder das weitere Bauteil (102) oder beide ein Cermet beinhaltet.

## Claims

1. A mechanical bearing (100) comprising a first component (101) and a further component (102),
wherein the mechanical bearing (100) is configured such that the first component (101) and the further component (102) can perform a bearing movement (103) against each other,
wherein the first member (101) or the further member (102) or both includes a cermet, wherein the cermet comprises at least 20% by weight of a metal based on the total weight of the cermet,
wherein the metal is biocompatible.

2. A mechanical bearing (100) comprising a first component (101) and a further component (102),
wherein the mechanical bearing (100) is configured such that the first component (101) and the further component (102) can perform a bearing movement (103) against each other,
wherein the first member (101) or the further member (102) or both includes a cermet, wherein the cermet comprises a metal to a metal moiety,
wherein the metal portion is a monotonously rising function from a position on a straight line passing through the cermet.

3. The mechanical bearing according to claim 2, wherein the metal content has a minimum in a range of 0 to 50% by weight based on the weight of a spatial section of the cermet.

4. The mechanical bearing according to any one of Claims 2 or 3, wherein the metal content has a maximum in a range of 50 to 95% by weight based on the weight of a spatial section of the cermet.

5. An implantable medical device (400) contains the mechanical bearing (100) according to one of the previous claims.

6. A process (600) comprising as process steps (601, 602):
a) Providing a housing; and
b) Inserting a moving part into the housing;
wherein the housing is made of a biocompatible material,
wherein said movable member is movably supported in said housing by said mechanical bearing (100) according to any one of claims 1 to 4.

7. A device available by the method (600) according to claim 6.

8. A use of a cermet for making a mechanical bearing (100),
wherein the cermet comprises at least 20% by weight of a metal based on the total weight of the cermet,
wherein the metal is biocompatible.

9. A use of a mechanical bearing (100) for supporting a component of an implantable medical device (400),
the mechanical bearing (100) comprising a first component (101) and a further component (102),
wherein the mechanical bearing (100) is configured such that the first component (101) and the further component (102) can perform a bearing movement (103) against each other, wherein the first member (101) or the further member (102) or both includes a cermet.

## Revendications

1. Palier mécanique (100) comprenant un premier composant (101) et un autre composant (102),
dans laquelle le palier mécanique (100) est configuré de telle sorte que le premier composant (101) et le composant supplémentaire (102) peuvent effectuer un mouvement de palier (103) l'un contre l'autre,
dans laquelle le premier élément (101) ou l'autre élément (102) ou les deux comprennent un cermet,
dans laquelle le cermet comprend au moins 20 % en poids d'un métal par rapport au poids total du cermet,
dans laquelle le métal est biocompatible.

2. Palier mécanique (100) comprenant un premier composant (101) et un autre composant (102),
dans laquelle le palier mécanique (100) est configuré de telle sorte que le premier composant (101) et le composant supplémentaire (102) peuvent effectuer un mouvement de palier (103) l'un contre l'autre,
dans laquelle le premier élément (101) ou l'autre élément (102) ou les deux comprennent un cermet,
dans laquelle le cermet comprend un fragment métal à métal,
dans laquelle la partie métallique est une fonction ascendante monotone à partir d'une position sur une ligne droite traversant le cermet.

3. Palier mécanique selon la revendication 2, dans lequel la teneur en métal a une valeur minimale comprise entre 0 et 50 % en poids par rapport au poids d'une section spatiale du cermet.

4. Palier mécanique selon l'une quelconque des revendications 2 ou 3, dans lequel la teneur en métal a un maximum dans une plage de 50 à 95 % en poids sur la base du poids d'une section spatiale du cermet.

5. Un dispositif médical implantable (400) contient le palier mécanique (100) selon l'une des revendications précédentes.

6. Procédé (600) comprenant comme étapes de procédé (601, 602) :
a) Fournir un logement ; et
b) Insertion d'une pièce mobile dans le boîtier ;
dans lequel le boîtier est fait d'un matériau biocompatible,
dans laquelle ledit élément mobile est supporté de manière mobile dans ledit logement par ledit palier mécanique (100) selon l'une quelconque des revendications 1 à 4.

7. Dispositif disponible par le procédé (600) selon la revendication 6.

8. Utilisation d'un cermet pour la fabrication d'un palier mécanique (100),
dans laquelle le cermet comprend au moins 20 % en poids d'un métal par rapport au poids total du cermet,
dans laquelle le métal est biocompatible.

9. Utilisation d'un palier mécanique (100) pour supporter un composant d'un dispositif médical implantable (400),
le palier mécanique (100) comprenant un premier composant (101) et un autre composant (102),
dans laquelle le palier mécanique (100) est configuré de telle sorte que le premier composant (101) et le composant supplémentaire (102) peuvent effectuer un mouvement de palier (103) l'un contre l'autre,
dans laquelle le premier élément (101) ou l'autre élément (102) ou les deux comprennent un cermet.
